# FASCICULE DE BREVET EUROPEEN

(11) **EP 0 708 100 B1**
(45) Date de publication et mention de la délivrance du brevet: **24.11.1999**
(21) Numéro de dépôt: 95402050.9
(22) Date de dépôt: 11.09.1995
(51) Int. Cl.: C07D 307/93, C07D 407/04, A61K 31/34, A61K 7/48, A61K 7/06

(54) **Nouveaux composés dérivés du dibenzofuranne, compositions pharmaceutiques et cosmétiques les contenant**
Neue, von Dibenzofuran abgeleitete Verbindungen und pharmazeutische und kosmetische Zusammensetzungen, die sie enthalten.
Dibenzofurane compounds, pharmaceutical and cosmetic compositions containing them

(30) Priorité: 04.10.1994 FR 9411853; 28.10.1994 FR 9412989
(43) Date de publication de la demande: 24.04.1996
(73) Titulaire: CENTRE INTERNATIONAL DE RECHERCHES DERMATOLOGIQUES GALDERMA, ( CIRD GALDERMA), 06560 Valbonne (FR)
(72) Inventeur: Charpentier, Bruno, F-06410 Biot (FR); Vion, Michèle, F-06130 Grasse Le Plan (FR); Bernard, Bruno, F-92200 Neuilly sur Seine (FR); Maignan, Jean, F-93290 Tremblay en France (FR)
(74) Mandataire: Tezier Herman, Béatrice

(56) Documents cités:
- EP-A- 0 360 637
- GB-A- 2 187 455

## Description

L'invention concerne, à titre de produits industriels nouveaux et utiles, des composés aromatiques polycycliques. Elle concerne également l'utilisation de ces nouveaux composés dans des compositions pharmaceutiques destinées à un usage en médecine humaine ou vétérinaire, ou bien encore dans des compositions cosmétiques.

Dans les documents EP-A-0360637 et GB-A-2187455 il a été décrit des composés benzofuranniques qui diffèrent de composés de formule (I) définie ci-après par leur structure chimique, et en particulier par leur noyau 2,3,4,4a-tétrahydro-4a,10,10-triméthyl-1H-3,9bméthano-dibenzofuranne de formule :

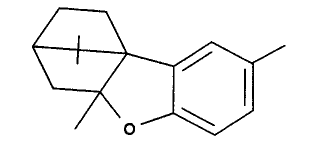

Les composés selon l'invention ont une activité marquée dans les domaines de la différenciation et de la prolifération cellulaire, et trouvent des applications plus particulièrement dans le traitement topique et systémique des affections dermatologiques liées à un désordre de la kératinisation, des affections dermatologiques (ou autres) à composante inflammatoire, virale et/ou immunoallergique, et des proliférations dermiques ou épidermiques qu'elles soient bénignes ou malignes. Ces composés peuvent en outre être utilisés dans le traitement des maladies de dégénérescence du tissu conjonctif, pour lutter contre le vieillissement de la peau, qu'il soit photoinduit ou chronologique, et traiter les troubles de la cicatrisation. Ils trouvent par ailleurs une application dans le domaine ophtalmologique, notamment dans le traitement des cornéopathies.

On peut également utiliser les composés selon l'invention dans des compositions cosmétiques, notamment pour l'hygiène corporelle et capillaire.

Les composés selon l'invention, dérivés du dibenzofuranne, sont caractérisés par le fait qu'ils présentent la formule générale (I) suivante : 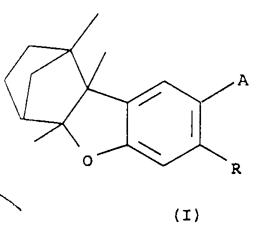 dans laquelle :
* R représente un atome d'hydrogène, un atome d'halogène, un radical alkyle inférieur, un radical acyle inférieur, un radical OR', R' ayant la signification donnée ci-après,
* A représente un radical choisi parmi les radicaux de formules (IIa-IIc) suivantes : 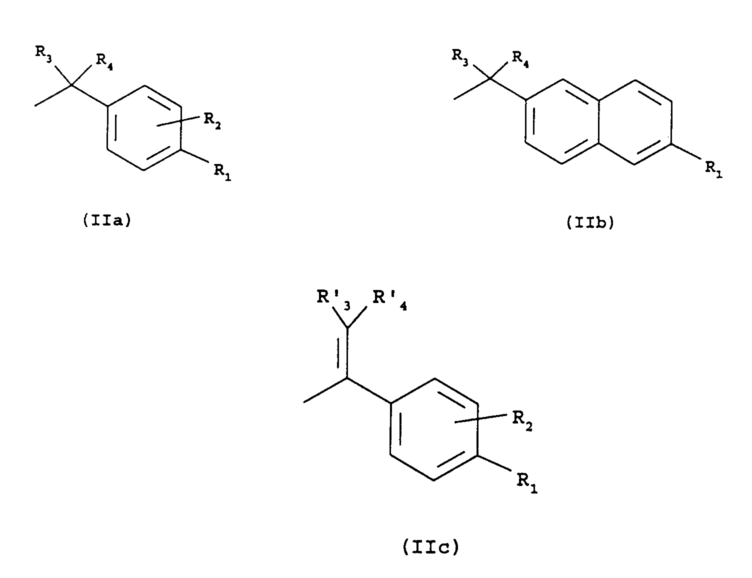 formules dans lesquelles :
   - R₁ représente :
      (i) un atome d'hydrogène,
      (ii) le radical -CH₃,
      (iii) le radical -CH₂-O-R_{5,}
      (iv) un radical -OR₅
      (v) un radical 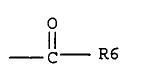
      (vi) un radical -S(O)ₜ R₆
      t, R₅ et R₆ ayant les signifcations données ci-après,
   - R₂ représente un atome d'hydrogène ou le radical -OR₅
      R₅ ayant la signification donnée ci-après,
   - soit R₃ et R₄ indépendamment représentent un atome d'hydrogène, un radical alkyle inférieur ou le radical -(X)ₙ - (CH₂)ₘ - R₇,
      X et R₇ ayant les significations données ci-après,
      soit R₃ et R₄, pris ensemble, forment un groupe oxo (=O), thiocetone (=S), oxime ou un dérivant de l'oxime (R₆-O-N=), epoxy, cycloalkyle éventuellement substitué par un atome d'halogène ou un radical alkyle inférieur, ou encore un groupe dioxolanne [-O-CH₂)_{q}-O-] avec q égal à 2 ou 3, R₆ ayant la signification donnée ci-après,
   - R₃' et R₄' représentent indépendamment un atome d'hydrogène, un radical alkyle inférieur, un radical acyle inférieur,
      étant entendu que dans tout ce qui précède :
      . t est égal à 0, 1 ou 2,
      . R₅ représentant un atome d'hydrogène, un radical alkyle inférieur, ou un radical acyle inférieur
      . R₆ représente :
         . un atome d'hydrogène,
         . un radical -N (R'',R'''), R'' et R''' ayant les significations données ci-après,
         . un radical -OR₈, R₈ ayant la signification donnée ci-après,
      . R₇ représente un atome d'hydrogène, un radical -(C0)ₚ-R₉ dans lequel p peut avoir la valeur 0 ou 1, R₉ ayant la signification donnée ci-après,
      . R₈ et R' représentent indépendamment un atome d'hydrogène, un radical alkyle ayant de 1 à 20 atomes de carbone, un radical mono ou polyhydroxyalkyle, un radical aryle éventuellement substitué, aralkyle éventuellement substitué, un reste de sucre ou un reste d'amino-acide,
      . R₉ représente :
         . un atome d'hydrogène,
         . un radical alkyle,
         . un radical alcenyle,
         . un radical alcynyle,
         . un radical aryle,
         . un radical -OR₇ - dans ce cas, m ne peut prendre la valeur O, R₇ ayant la signification indiquée ci-dessus.
         . un radical -N(R'',R''), R'' et R''' ayant les significations données ci-après,
      . R'' et R''', identiques ou différents, représentent un atome d'hydrogène, un radical alkyle inférieur, un radical mono ou polyhydroxyalkyle, un radical aryle éventuellement substitué, un radical aralkyle éventuellement substitué, un reste d'amino-acide ou de peptide ou de sucre ou encore R'' et R''' pris ensemble forment un hétérocycle,
      . X est un atome d'oxygène ou de soufre,
      . n varie de 0 à 1 et m de 0 à 10,
et les isomères optiques et géométriques des dits composés de formule (I), ainsi que leurs sels dans le cas où R₁ ou R₉ représente une fonction acide, ou lorsque R₉ représente une fonction amine.

Lorsque les composés selon l'invention se présentent sous forme de sels par addition d'une base, il s'agit de préférence de sels d'un métal alcalin ou alcalino-terreux ou encore de zinc ou d'une amine organique.

Lorsque les composés se présentent sous forme de sels, par addition d'un acide, il s'agit de sels pharmaceutiquement ou cosmétiquement acceptables obtenus par addition d'un acide minéral ou organique, en particulier l'acide chlorhydrique, sulfurique, acétique, citrique, fumarique, hémisuccinique, maléique et mandélique.

Par radical alkyle inférieur, on entend un radical ayant de 1 à 6 atomes de carbone et de préférence les radicaux méthyle, éthyle, isopropyle, butyle, tertiobutyle et hexyle.

Par radical alcényle, on entend un radical ayant de 1 à 20 atomes de carbones, linéaire ou ramifié présentant une double liaison.

Par radical alcynyle, on entend un radical ayant de 1 à 20 atomes de carbone, linéaire ou ramifié présentant une triple liaison.

Par radical acyle inférieur, on entend un radical ayant de 1 à 10 atomes de carbone et de préférence les radicaux acétyle, propionyle, pivaloyle.

Les radicaux alkyle ayant de 1 à 20 atomes de carbone sont linéaires ou ramifiés, éventuellement substitués par 1 ou plusieurs atomes de fluor.

Par radical monohydroxyalkyle, on entend un radical ayant de 1 à 6 atomes de carbone, notamment un radical 2-hydroxyéthyle, 2-hydroxypropyle ou 3-hydroxypropyle.

Par radical polyhydroxyalkyle, on entend un radical contenant de 2 à 6 atomes de carbone et de 2 à 5 groupes hydroxyles, tels que les radicaux 2,3-dihydroxypropyle, 2,3,4-trihydroxybutyle, 2,3,4,5-tétrahydroxypentyle ou le reste du pentaérythritol.

Parmi les radicaux aryle, on choisit plus particulièrement un radical phényle éventuellement substitué par un ou plusieurs atomes d'halogène, une fonction hydroxyle, nitro ou un groupe méthoxy.

Par radical aralkyle éventuellement substitué, on entend le radical benzyle ou phénéthyle, éventuellement substitué par un ou plusieurs atomes d'halogène, une fonction hydroxyle ou nitro, ou un groupe méthoxy.

Par reste d'amino-acide, on entend un reste dérivant par exemple de l'un des 20 aminoacides de configuration L ou D constitutifs de protéines de mammifères.

Par reste de peptide, on entend un peptide linéaire de 2 à 10 acides aminés.

Par reste de sucre, on entend un reste dérivant par exemple du glucose, du galactose, du mannose ou de l'acide glucuronique.

Les hétérocycles préférés correspondent au radical pipéridino, morpholino, pyrrolidino ou pipérazino, éventuellement substitué en position 4 par un radical alkyle en C₁-C₆ ou mono ou polyhydroxyalkyle tels que définis ci-dessus.

Parmi les composés de formule générale (I), on peut notamment citer les suivants :
[(1,2,3,4-Tétrahydro-1,4a,9b-triméthyl-1,4-méthanodibenzofuranne-8-yl)carbonyl]-6-naphtalène-2-carboxylate de méthyle
Acide [(1,2,3,4-Tétrahydro-1,4a,9b-triméthyl-1,4-méthano-dibenzofuranne-8-yl) carbonyl]-6-naphtalène-2-carboxylique
Acide [(1,2,3,4-Tétrahydro-1,4a,9b-triméthyl-1,4-méthano-dibenzofuranne-8-yl) méthyl]-6-naphtalène-2-carboxylique
[(1,2,3,4-Tétrahydro-1,4a,9b-triméthyl-1,4-méthanodibenzofuranne-8-yl) hydroxyméthyl]-6-naphtalène-2-carboxylate de méthyle
Acide [(1,2,3,4-Tétrahydro-1,4a,9b-triméthyl-1,4-méthano-dibenzofuranne-8-yl) hydroxyméthyl]-6-naphtalène-2-carboxylique
4-[(1,2,3,4-Tétrahydro-1,4a,9b-triméthyl-1,4-méthanodibenzofuranne-8-yl)carbonyl) benzoate de méthyle
Acide 4-[(1,2,3,4-Tétrahydro-1,4a,9b-triméthyl-1,4-méthano-dibenzofuranne-8-yl)carbonyl] benzoïque
4-[(1,2,3,4-Tétrahydro-1,4a,9b-triméthyl-1,4-méthanodibenzofuranne-8-yl) hydroxyméthyl] phénylcarbinol
4-[(1,2,3,4-Tétrahydro-1,4a,9b-triméthyl-1,4-méthanodibenzofuranne-8-yl)carbonyl] phénylcarboxaldéhyde
2-(1,2,3,4-Tétrahydro-1,4a,9b-triméthyl-1,4-méthanodibenzofuranne-8-yl)-2-(4-méthoxycarbonylphényl)-1,3-dioxolanne
2-(1,2,3,4-Tétrahydro-1,4a,9b-triméthyl-1,4-méthanodibenzofuranne-8-yl)-2-(4-carboxyphényl)-1,3-dioxolanne
4-[(1,2,3,4-tetrahydro-1,4a,7,9b-tetraméthyl-1,4-methanodibenzofuranne-8-yl) carbonyl] benzoate de méthyle
Acide 4-[(1,2,3,4-tetrahydro-1,4a,7,9b-tetraméthyl-1,4-methanodibenzofuranne-8-yl) carbonyl] benzoïque.

Parmi les composés selon l'invention, on préfère les composés répondant à la formule générale (I) dans laquelle A représente le radical de formule (IIb).

Les composés plus particulièrement préférés sont ceux pour lesquels A représente le radical de formule (IIb) dans laquelle R₁ représente le radical

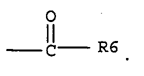

La présente invention a également pour objet, à titre de produits intermédiaires nouveaux, les composés correspondant à la formule générale (X) suivante : 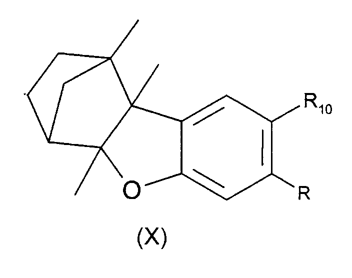 formule (X) dans laquelle R a les signifcations de R des composés de formule (I), R₁₀ peut avoir toutes les significations de R₁ précédemment décrit, à l'exception de l'atome d'hydrogène, ou peut également représenter un atome d'halogène, le radical -C(O)-R₈, ou le radical -C(O)-CH₂-halogène ou (CH₂)ₙ N (R''R'''), R₈ , R'' et R''' étant tels que définis dans la formule générale (I).

Parmi les composés de formule (X) ci-dessus, on peut notamment citer les suivants :
1,2,3,4-Tétrahydro-1,4a,9b-triméthyl-1,4-méthanodibenzofuranne-8-yl méthylcétone
8-(1,2,3,4-Tétrahydro-1,4a,9b-triméthyl-1,4-méthanodibenzofuranne)carboxaldéhyde
Acide 8-(1,2,3,4-Tétrahydro-1,4a,9b-triméthyl-1,4-méthanodibenzofuranne) carboxylique
1,2,3,4-Tétrahydro-1,4a,9b-triméthyl-1,4-méthanodibenzofuranne-8-yl, bromométhylcétone
8-Ethynyl- 1,2,3,4-tétrahydro-1,4a,9b-triméthyl-1,4-méthanodibenzofuranne
1,2,3,4-Tetrahydro-1,4a,7,9b-tetraméthyl-1,4-methanodibenzofuranne

La présente invention a également pour objet les procédés de préparation des composés de formule (I) qui sont les suivants :
- soit par réaction de Friedel et Crafts entre le 1,2,3,4-Tétrahydro-1,4a,9b-triméthyl-1,4-méthanodibenzofuranne ou un dérivé de ce noyau substitué de formule (III) (famille des composés de formule (X)) et un chlorure d'acide de formule générale (IV) ou (V) (voir shéma réactionnel ci-dessous).

Le 1,2,3,4-Tétrahydro-1,4a,9b-triméthyl-1,4-méthanodibenzofuranne (THTMDBF) est obtenu en 2 étapes : par addition du o-anisyllithium sur la Fenchone, suivie de l'action du pentachlorure de phosphore, du tribromure de phosphore ou d'un acide de lewis. La Fenchone peut être dextrogyre ou lévogyre.

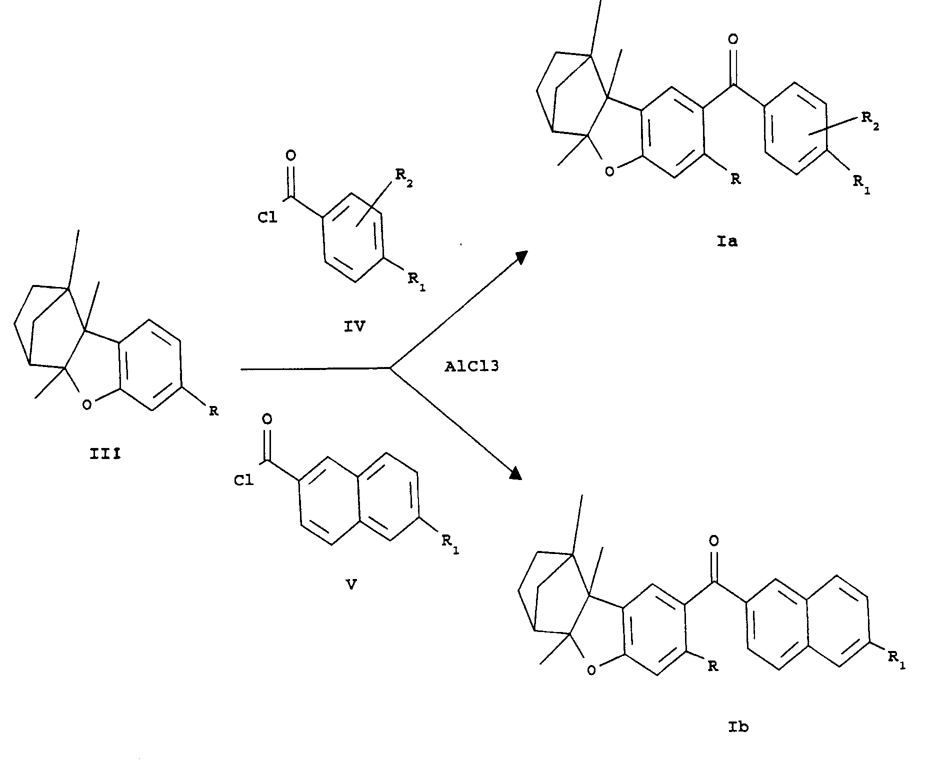

Les formules Ia et Ib correspondent à la formule (I) dans laquelle A représente respectivement les radicaux de formules IIa et IIb, dans lesquelles R₃ et R₄ pris ensemble forment un groupe oxc.
- soit par réaction d'un dérivé organométallique en position -8 du noyau (formule III), tel qu'un organozincique ou un organostannane (formule VII), sur un composé de formules IV ou V, catalysée par un dérivé du palladium selon le schéma ci-après, 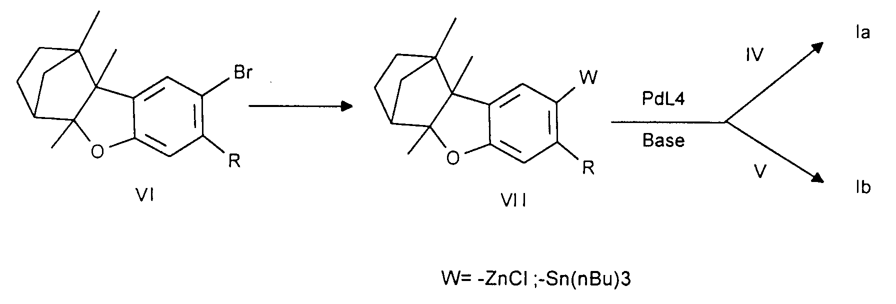
- soit dans des conditions de carbonylation :

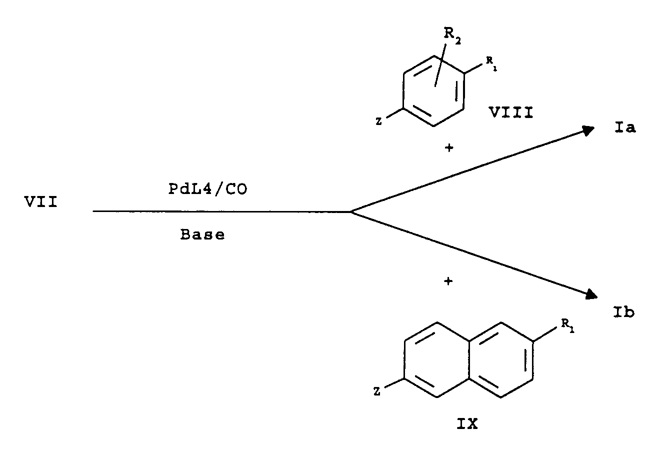

Dans les formules (VIII) et (IX), Z représente Br, l ou O-SO₂-CF₃

Au cours de ces réactions conduisant aux composés de formule générale (I), les fonctions R₁ et R₂ seront éventuellement sous une forme protégée pour être compatibles avec les conditions opératoires. Les groupements protecteurs employés sont ceux décrits dans le livre "Protecting Groups in Organic Synthesis" by T.W. Greene, Ed. by John Wiley and Sons (1981).

Les dérivés (Ia) et (Ib) servent ensuite de produit de départ pour la fabrication d'autres composés selon les méthodes classiques de la chimie organique telles que celles décrites dans le livre "Advanced Organic Chemistry" de J. March; John Wiley and sons, 1985. En particulier, la modification de la fonction carbonyle conduira aux différentes significations de R₃ et R₄.

Ces composés présentent une activité agoniste ou antagoniste partielle vis-à-vis de l'expression d'un ou plusieurs marqueurs biologiques dans le test de différenciation des cellules (F9) de tératocarcinome embryonnaire de la souris (Skin Pharmacol. 3, p.256-267, 1990) et/ou sur la différenciation des kératinocytes humains in vitro (Skin Pharmacol. 3 p.70-85, 1990) en réponse à un traitement par des rétinoïdes. Ces tests sus-mentionnés montrent les activités des composés dans les domaines de la différenciation et de la prolifération. Leurs activités peuvent aussi être mesurées dans des tests de transactivation cellulaire à l'aide de récepteurs recombinants RARs ou RXRs préalablement transfectés. (B.A. Bernard et al., Biochemical and Biophysical Research Communication 1992, vol. 186, 977-983; M.F. Boehm et al. Journal of Medicinal Chemistry, 1994, 37, 408-414).

La présente invention a également pour objet à titre de médicament les composés de formule (I) tels que décrits ci-dessus.

Les composés selon l'invention conviennent particulièrement bien dans les domaines de traitement suivants :
1) Pour traiter les affections dermatologiques liées à un désordre de la kératinisation portant sur la différenciation et sur la prolifération notamment pour traiter les acnés vulgaires, comédoniennes, polymorphes, rosacées, les acnés nodulokystiques, conglobata, les acnés séniles, les acnés secondaires telles que l'acné solaire, médicamenteuse ou professionnelle.
2) Pour traiter d'autres types de trouble de la kératinisation, notamment les ichtyoses, les états ichtyosiformes, la maladie de Darier, les kératodermies palmoplantaires, les leucoplasies et les états leucoplasiformes, le lichen cutané ou muqueux (buccal).
3) Pour traiter d'autres affections dermatologiques liées à un trouble de la kératinisation avec une composante inflammatoire et/ou immuno-allergique et, notamment, toutes les formes de psoriasis qu'il soit cutané, muqueux ou unguéal, et même le rhumatisme psoriatique, ou encore l'atopie cutanée, telle que l'eczéma ou l'atopie respiratoire ou encore l'hypertrophie gingivale; les composés peuvent également être utilisés dans certaines affections inflammatoires ne présentant pas de trouble de la kératinisation.
4) Pour traiter toutes les proliférations dermiques ou épidermiques qu'elles soient bénignes ou malignes, qu'elles soient ou non d'origine virale telles que verrues vulgaires, les verrues planes et l'épidermodysplasie verruciforme, les papillomatoses orales ou florides et les proliférations pouvant être induites par les ultra-violets notamment dans le cas des épithélioma baso et spinocellulaires.
5) Pour traiter d'autres désordres dermatologiques tels que les dermatoses bulleuses et les maladies du collagène.
6) Pour traiter certains troubles ophtalmologiques, notamment les cornéopathies.
7) Pour réparer ou lutter contre le vieillissement de la peau, qu'il soit photoinduit ou chronologique ou pour réduire les pigmentations et les kératoses actiniques, ou toutes pathologies associées au vieillissement chronologique ou actinique.
8) Pour prévenir ou guérir les stigmates de l'atrophie épidermique et/ou dermique induite par les corticostéroïdes locaux ou systémiques, ou tout autre forme d'atrophie cutanée.
9) Pour prévenir ou traiter les troubles de la cicatrisation ou pour prévenir ou réparer les vergetures.
10) Pour lutter contre les troubles de la fonction sébacée tels que l'hyperséborrhée de l'acné ou la séborrhée simple.
11) Dans le traitement ou la prévention des états cancéreux ou précancéreux.
12) Dans le traitement d'affections inflammatoires telles que l'arthrite.
13) Dans le traitement de toute affection d'origine virale au niveau cutané ou général.
14) Dans la prévention ou le traitement de l'alopécie.
15) Dans le traitement d'affections dermatologiques ou générales à composante immunologique.
16) Dans le traitement d'affections du système cardiovasculaire telles que l'artériosclérose.
17) Dans le traitement d'affections respiratoires.

Dans les domaines thérapeutiques mentionnés ci-dessus, les composés selon l'invention peuvent être avantageusement employés en combinaison avec d'autres rétinoïdes, avec des ligands des récepteurs RXR, avec des dérivés de la vitamine D, avec des corticostéroïdes ou des estrogènes, en association avec des anti-oxydants, avec des α-hydroxy ou α-céto acides ou leurs dérivés, ou encore avec des bloqueurs de canaux potassiques.

Parmi les ligands des récepteurs RXR, on peut citer l'acide rétinoïque 9-cis.

Parmi les vitamines D ou leurs dérivés, on peut notamment citer les dérivés de la vitamine D₂ ou D₃ et en particulier la 1,25-dihydroxyvitamine D₃.

Parmi les antiradicaux libres, on peut notamment citer l'α-tocophérol, la Super Oxyde Dismutase, l'Ubiquinol ou certains chélatants de métaux.

Parmi les α-hydroxy ou α-céto acides ou leurs dérivés, on peut notamment citer les acides lactique, malique, citrique, glycolique, mandélique, tartrique, glycérique, ascorbique ou dérivés d'acide salicylique ou leurs sels, amides ou esters.

Parmi les bloqueurs de canaux potassiques, on peut notamment citer le Minoxidil (2,4-diamino-6-pipéridino-pyrimidine-3-oxyde) et ses dérivés.

La présente invention a donc aussi pour objet une nouvelle composition médicamenteuse destinée notamment au traitement des affections susmentionées, caractérisée par le fait qu'elle comporte, dans un support pharmaceutiquement acceptable au moins un composé de formule (I), l'un de ses isomères optiques ou géométriques ou un de ses sels.

L'administration des composés de formule (I) selon l'invention peut être effectuée par voie entérale, parentérale, topique ou oculaire.

Par voie entérale, les médicaments peuvent se présenter sous formes de comprimés, de gélules, de dragées, de sirops, de suspensions, de solutions, de poudres, de granulés, d'émulsions, de microsphères ou nanosphères ou vésicules lipidiques ou polymériques permettant une libération contrôlée. Par voie parentérale, les compositions peuvent se présenter sous forme de solutions ou suspensions pour perfusion ou pour injection.

Les composés de formule (I) selon l'invention sont généralement administrés à une dose journalière d'environ 0,01 mg/kg à 100 mg/kg en poids corporel en 1 à 3 prises.

Par voie topique, les compositions pharmaceutiques à base de composés selon l'invention sont destinées au traitement de la peau et des muqueuses et se présentent sous forme d'onguents, de crèmes, de laits, de pommades, de poudres, de tampons imbibés, de solutions, de gels, de sprays, de lotions ou de suspensions. Elles peuvent également se présenter sous forme de microsphères ou nanosphères ou vésicules lipidiques ou polymériques ou de patches polymériques et d'hydrogels permettant une libération contrôlée. Ces compositions par voie topique peuvent se présenter soit sous forme anhydre, soit sous forme aqueuse selon l'indication clinique.

Par voie oculaire, ce sont principalement des collyres.

Ces compositions pour la voie topique ou oculaire contiennent au moins un composé de formule (I) telle que définie ci-dessus, l'un de ses isomères optiques ou géométriques ou un de ses sels, à une concentration de préférence comprise entre 0,001 et 5 % par rapport au poids total de la composition.

Les composés de formule (I), selon l'invention, trouvent également une application dans le domaine cosmétique, en particulier dans l'hygiène corporelle et capillaire et notamment pour le traitement des peaux à tendance acnéique, pour la repousse des cheveux, l'anti-chute, pour lutter contre l'aspect gras de la peau ou des cheveux, dans la protection contre les effets néfastes du soleil ou dans le traitement des peaux physiologiquement sèches, pour prévenir et/ou lutter contre le vieillissement photo-induit ou chronologique.

Dans le domaine cosmétique, les composés de formule (I) selon l'invention peuvent être avantageusement employés en combinaison avec d'autres rétinoïdes, avec les vitamines D ou leurs dérivés, avec des corticostéroïdes, en association avec des anti-radicaux libres, avec des α-hydroxy ou α-céto acides ou leurs dérivés, ou encore avec des bloqueurs de canaux ioniques. Ces différents produits employés avec les composés de la présente invention étant tels que définis ci-dessus.

La présente invention vise donc également une composition cosmétique comprenant, dans un support cosmétiquement acceptable, au moins un composé de formule (I), l'un de ses isomères optiques ou géométrique ou l'un de ses sels. Cette composition peut se présenter notamment sous forme d'une crème, d'un lait, d'une lotion, d'un gel, de microsphères ou nanosphères ou vésicules lipidiques ou polymériques, d'un savon ou d'un shampooing.

La concentration en composé de formule (I), dans les compositions cosmétiques est comprise entre 0,001 et 3 % en poids.

Les compositions médicamenteuses et cosmétiques selon l'invention peuvent, en outre, contenir des additifs inertes ou même pharmacodynamiquement ou cosmétiquement actifs ou des combinaisons de ces additifs et notamment : des agents mouillants; des agents dépigmentants tels que l'hydroquinone, l'acide azelaïque, l'acide caféïque ou l'acide kojique; des émollients; des agents hydratants comme le glycérol, le PEG 400, la thiamorpholinone et ses dérivés ou l'urée; des agents antiséborrhéiques ou antiacnéiques, tels que la S-carboxyméthylcystéine, la S-benzyl-cystéamine, leurs sels et leurs dérivés, ou le péroxyde de benzoyle; des antibiotiques comme l'érythromycine et ses esters, la néomycine, la clindamycine et ses esters, les tétracyclines; des agents antifongiques tels que le kétoconazole ou les polyméthylène-4,5 isothiazolinones-3 ; des agents favorisant la repousse des cheveux, comme le Minoxidil (2,4-diamino-6-pipéridino-pyrimidine-3-oxyde) et ses dérivés, le Diazoxide (7-chloro 3-méthyl 1,2,4-benzothiadiazine 1,1-dioxyde) et le Phénytoïn (5,4-diphényl-imidazolidine 2,4-dione) ; des agents anti-inflammatoires non stéroïdiens; des caroténoïdes et, notamment le b-carotène; des agents anti-psoriatiques tels que l'anthraline et ses dérivés et enfin, les acides eicosa-5,8,11,14- tétraynoïque et eicosa-5,8,11-trynoïque, leurs esters et les amides.

Les compositions selon l'invention peuvent également contenir des agents d'amélioration de la saveur, des agents conservateurs tels que les esters de l'acide parahydroxybenzoïque, les agents stabilisants, des agents régulateurs d'humidité, des agents régulateurs de pH, des agents modificateurs de pression osmotique, des agents émulsionnants, des filtres UV-A et UV-B, des anti-oxydants, tels que l'a-tocophérol, le butylhydroxyanisole ou le butylhydroxytoluène.

On va maintenant donner, à titre d'illustration et sans aucun caractère limitatif, plusieurs exemples de préparation des composés actifs de formule (I) selon l'invention, ainsi que des exemples de compositions les contenant.

### A. EXEMPLES DE COMPOSES

Tous les produits dont les synthèses sont exposées ci-après ont été caractérisés par RMN du proton (250 MHz), spectrométrie masse et analyse élementaire.

### Exemple 1:

### 1,2,3,4-Tetrahydro-1,4a,9b-triméthyl-1,4-methanodibenzofuranne:

50 g (0,267 moles) de bromoanisole en solution dans 120 ml d'ether éthylique sont traités à 0°C par 200 ml de n-Butylithium (1,6 M dans l'hexane), puis le milieu est laissé sous agitation à température ambiante durant la nuit. On ajoute alors goutte à goutte 41,57 g (273 mmoles) de (+) Fenchone (Fluka) dans 100 ml d'éther éthylique et laisse sous agitation 6 h à température ambiante. Le milieu réactionnel est versé dans 200 ml d'une solution saturée de chlorure d'ammonium.
Après extraction par 600 ml d'éther éthylique, rinçage à l'eau, séchage sur sulfate de magnésium, filtration et évaporation, le résidu est chromatographié sur silice pour conduire à 61,26 g (88%) de l'intermédiaire attendu fondant à 62-64°C.

### Méthode A

A une suspension de 55,24 g (0.21 moles) de cet intermédiaire et de 4 g de carbonate de calcium dans 800 ml de chloroforme, on ajoute à -10°C, 57,5 g (0,276 moles) de pentachlorure de phosphore.

Le mélange réactionnel est agité à température ambiante durant deux heures, puis on ajoute du carbonate de potassium (30 g) et on filtre. Le résidu solide est rincé au chloroforme puis chromatographié sur silice dans un mélange hexane/CH₂Cl₂ (9:1) pour conduire à 31,5g (65%) du dérivé attendu fondant à 68°C.

La même synthèse effectuée à partir de la (-) fenchone conduit à l'isomère dextrogyre du 1,2,3,4-Tétrahydro-1,4a, 9b-triméthyl-1,4-méthano dibenzo furanne, fondant à 68°C.

### Méthode B

A une solution de 55,24 g (0.21 moles) de cet intermédiaire dans 800 ml de dichlorométhane, on ajoute à 0°C, 28,6 g de chlorure de zinc.

Le mélange réactionnel est agité à température ambiante durant une nuit puis est versé dans 500 ml d'eau glacé, puis ce milieu est extrait par 500 ml d'ether. Après rinçage à l'eau, séchage sur sulfate de magnésium et évaporation, on isole parès chromatographie. Le résidu solide est rincé au chloroforme puis chromatographié sur silice dans un mélange hexane/CH₂Cl₂ (9:1) pour conduire à 19,8g (41%) du dérivé attendu.

### Exemple 2 :

### 8-Bromo-(1,2,3,4-tetrahydro-1,4a,9b-triméthyl-1-1,4-methanodibenzofuranne

11,42 g (50 mmoles) de (-)(1,2,3,4-Tetrahydro-1-4a,9b-triméthyl-1,4-methanodibenzofuranne) obtenus à l'exemple 1 sont dissous dans 110 ml de tetrahydrofuranne et sont traités goutte à goutte par une solution contenant 8,9 g de N-Bromosuccinimide (NBS) dans 50 ml de diméthylformamide (DMF). On laisse agiter à température ambiante pendant 2 h 30 puis verse le milieu réactionnel dans de l'eau glacée et extrait par 500 ml d'éther éthylique. Après rincage à l'eau, séchage sur sulfate de magnésium, filtration et évaporation, on isole après chromatographie sur silice dans l'hexane 13,8g (90%) du dérivé attendu fondant à 119,8 °C.

### Exemple 3 :

### [(1,2,3,4-tetrahydro-1,4a,9b-triméthyl-1,4-methanodibenzofuranne-8-yl) carbonyl]-6-naphtalène-2-carboxylate de méthyle

A une suspension de 5,22 g (39,1 mmoles) de chlorure d'aluminium dans 100 ml de dichlorométhane anhydre, on ajoute goutte à goutte une solution de 5,96 g (26,1 mmoles) du dérivé obtenu à l'exemple 1 et 6,49 g (26,1 mmoles) de chlorure de l'acide 6-méthoxycarbonyl-2-naphtalènecarboxylique dans 100 ml de dichlorométhane. Le mélange est agité pendant 4 heures à température ambiante, puis versé dans de l'eau glacée. Après décantation, la phase aqueuse est extraite par 500 ml de CH₂Cl₂. Les phases organiques sont rincées à l'eau, séchées sur sulfate de magnésium et évaporées. On isole après chromatographie sur silice dans le mélange CH₂Cl₂/hexane (9:1) 7,53 g (65%) du dérivé attendu fondant à 146°C.

### Exemple 4 :

### Acide[(1,2,3,4-tetrahydro-1,4a,9b-triméthyl-1,4-methanodibenzofuranne-8-yl)carbonyl]-6-naphtalène-2-carboxylique.

7,52 g (17 mmoles) de l'ester méthylique obtenu à l'exemple 3 en solution dans 100 ml de méthanol sont traités par 8 g de soude et chauffés à reflux 3 h 30. Après évaporation, le résidu est repris dans l'eau et acidifié à pH1 avec de l'acide chlorydrique concentré. Le précipité est filtré, rincé à l'eau, puis est recristallisé dans le méthanol pour conduire à 5,16 g (82%) du dérivé attendu fondant à 269,7°C.

### Exemple 5 :

### Acide[(1,2,3,4-tetrahydro-1,4a,9b-triméthyl-1,4-methanodibenzofuranne-8-yl)methyl]-6-naphtalène-2-carboxylique

0,5 g (1,17 mmoles) de l'acide obtenu à l'exemple 4 sont placés dans 50 ml de palladium (10%) sur charbon sous une pression de 7 bars d'hydrogène pendant 24 heures. Le milieu réactionnel est filtré sur célite puis évaporé. Le résidu est chromatographié sur silice dans le mélange CH₂Cl₂/ether ethylique (9:1) pour conduire à 110 mg (23%) du dérivé attendu fondant à 214-216°C.

### Exemple 6 :

### [(1,2,3,4-tetrahydro-1,4a,9b-triméthyl-1,4-methanodibenzofuranne-8-yl)méthanol]-6-naphtalène-2-carboxylate de méthyle

944 mg (2,14 mmoles) de l'ester obtenu à l'exemple 3 dissous dans 30 ml d'un mélange méthanol/tetrahydrofuranne (1:1), sont traités par 81 mg de borohydrure de sodium. Le milieu réactionnel est agité 5 heures à température ambiante. On évapore à sec, reprend dans l'éther et lave cette phase jusqu'à pH neutre. Après séchage et évaporation, on isole 905 mg (96%) du dérivé attendu fondant à 140-141°C.

### Exemple 7 :

### Acide[(1,2,3,4-tetrahydro-1,4a,9b-triméthyl-1,4-methanodibenzofuranne-8-yl)methanol]-6-naphtalène-2-carboxylique.

898 mg (2,03 mmoles) de l'ester obtenu à l'exemple 6 dans 25 ml de méthanol sont traités par 2 g de soude. Le milieu réactionnel est laissé sous agitation durant 48 heures. Le milieu réactionnel est versé dans l'eau glacée, acidifié à pH1 avec de l'acide chlorydrique concentré et extrait par de l'acétate d'éthyle. La phase organique est lavée à l'eau, séchée sur sulfate de magnésium, filtrée et évaporée pour conduire à 951 mg (92%) du dérivé attendu fondant à 146-148°C.

### Exemple 8

### 4-[(1,2,3,4-tetrahydro-1,4a,9b-triméthyl-1,4-methanodibenzofuranne-8-yl) carbonyl] benzoate de méthyle

A une solution composée de 6,76 g (29,6 mmoles) de (+) THTMDBF et 5,72 g (28,8 mmoles) de chlorure d'acide du monotéréphtalate de méthyle dans 130 ml de CH₂Cl₂, on ajoute goutte à goutte 5,92 g (44 mmoles) de chlorure d'aluminium et laisse agiter à température ambiante durant la nuit.
Après le même traitement qu' à l'exemple 3, suivi d'une chromatographie sur silice dans le mélange d'éluant CH₂Cl₂/hexane (60:40), on isole 4,47 g (40%) du dérivé attendu fondant à 150°C.

### Exemple 9:

### Acide 4-[(1,2,3,4-tetrahydro-1,4a, 9b-triméthyl-1,4-methanodibenzofuranne-8-yl) carbonyl] benzoïque

2,52 g (6,44 mmoles) de l'ester obtenu à l'exemple 8 en solution dans 30 ml de méthanol sont traités par 2,5 g de soude et chauffés à reflux pendant 3 heures. Après le même traitement qu'à l'exemple 7, suivi d'une recristallisation dans l'hexane, on isole 2,35 g (97%) du produit attendu fondant à 262-264°C.

### Exemple 10 :

### 4-[(1,2,3,4-tetrahydro-1,4a,9b-triméthyl-1,4-methanodibenzofuranne-8-yl) hydroxymethyl] phenylcarbinol

A une suspension de 1,14 g d'hydrure de Lithium aluminium dans 10 ml de tetrahydrofuranne, on ajoute goutte à goutte une solution de 3,76 g (10 mmoles) de l'acide obtenu à l'exemple 9 et laisse agiter 3 heures à température ambiante. On neutralise à 0°C le milieu réactionnel par addition goutte à goutte d'une solution saturée de chlorure d'ammonium. Le précipité est filtré, lavé par de l'hexane et séché pour conduire à 3,13 g (86%) du dérivé attendu fondant 113 - 115 °C.

### Exemple 11 :

### 4-[(1,2,4-tetrahydro-1,4a,9b-triméthyl-1,4-methanodibenzofuranne -8-yl) carbonyl] phenylcarboxaldéhyde

3,1 g (8,5 mmoles) du diol obtenu à l'exemple 10 en solution dans 60 ml de CH₂Cl₂ sont traités par 5,5 g de Pyridinium chlorochromate. La réaction est laissée sous agitation à température ambiante pendant 3 heures. Le milieu réactionnel est alors filtré sur célite. La phase organique est lavée avec une solution saturée de chlorure d'ammonium, rincée à l'eau, séchée et évaporée pour conduire après chromatographie sur silice dans le mélange d'éluant CH₂Cl₂/hexane (9:1) à 2 g (66%) du dérivé attendu fondant à 138-142°C.

### Exemple 12 :

### 2-(1,2,3,4-tetrahydro-1,4a,9b-triméthyl-1,4-methanodibenzofuranne -8yl),2-(4-methoxycarbonylphenyl)-1,3-dioxolanne

2 g (5,12 mmoles) de la cétone obtenu à l'exemple 8 dans 20 ml de benzène sont traités par 0,5 ml d'éthylène glycol et 10 mg de TSOH. Le milieu réactionnel est chauffé à reflux pendant 24 heures, puis est neutralisé par une solution saturée de bicarbonate de sodium. Après extraction par de l'éther éthylique, la phase organique est lavée à l'eau, séchée sur sulfate de magnésium, filtrée et évaporée pour conduire après chromatographie sur silice dans le mélange Hexane/AcoEt (90:10) et empatage dans l'hexane, à 910 mg (41%) du dérivé attendu fondant à 136-137°C.

### Exemple 13 :

### 2-(1,2,3,4-tetrahydro-1,4a,9b-triméthyl-1,4-methanodibenzofuranne -8-yl),2-(4-carboxyphenyl)-1,3-dioxolanne

0,87 g (2 mmoles) de l'ester méthylique obtenu à l'exemple 12 sont saponifiés dans les conditions décrites à l'exemple 4. On recueille après le même traitement et recristallisation dans l'ester diisopropryl, 0,62 g (74%) du suivi attendu fondant à 234-236°C.

### Exemple 14 :

### 1,2,3,4-Tetrahydro-1,4a,7,9b-tetraméthyl-1,4-methanodibenzofuranne

12,7 g (63 mmoles) de 2-methoxy-4-bromobenzène en solution dans 30 ml d'éther éthylique sont traités à 0°C par 47.5 ml de Butylithium (1,6 M dans l'hexane), puis le milieu est laissé sous agitation à température ambiante durant la nuit. On ajoute alors goutte à goutte 47,5 g (76 mmoles) de (+) Fenchone (Fluka) dans 100 ml d'éther éthylique et laisse sous agitation 6 h à température ambiante. Le milieu réactionnel est versé dans 200 ml d'une solution saturée de chlorure d'ammonium.

Après extraction par 400 ml d'éther éthylique, rinçage à l'eau, séchage sur sulfate de magnésium, filtration et évaporation, le résidu est chromatographié sur silice pour conduire à 8,96 g (82 %) de l'intermédiaire attendu fondant à 102-103°C.

A une suspension de 5,48 g (21 mmoles) de cet intermédiaire et de 2,8 g de carbonate de calcium dans 50 ml de chloroforme, on ajoute à -10°C 5,41 g (26 mmoles) de pentachlorure de phosphore.

Le mélange réactionnel est agité à température ambiante durant deux heures, puis on ajoute du carbonate de potassium et on filtre. Le résidu solide est rincé au chloroforme puis chromatographié sur silice dans un mélange hexane/CH₂Cl₂ (9:1) pour conduire à 2,75 g (57 %) du dérivé attendu sous forme d'huile incolore.

### Exemple 15 :

### 4-[(1,2,3,4-tetrahydro-1,4a,7,9b-tetraméthyl-1,4-methanodibenzofuranne-8-yl) carbonyl] benzoate de méthyle

A une solution composée de 1,7 g (7,1 mmoles) du dérivé obtenu à l'exemple 14 et 1,42 g (7,16 mmoles) de chlorure d'acide du monotéréphtalate de méthyle dans 30 ml de CH₂Cl₂, on ajoute goutte à goutte 1,43 g (10,7 mmoles) de chlorure d'aluminium et laisse agiter à température ambiante durant la nuit.

Après le même traitement qu'à l'exemple 3, suivi d'une chromatographie sur silice dans le mélange d'éluant CH₂Cl₂/heptane (70:30), on isole 0,8 g (29 %) du dérivé attendu fondant à 140-142°C; αD= -6° ( c=1, CHCl₃).

### Exemple 16 :

### Acide 4-[(1,2,3,4-tetrahydro-1,4a,7,9b-tetraméthyl-1,4-methanodibenzofuranne-8-yl) carbonyl] benzoïque

0,83 g (2 mmoles) de l'ester obtenu à l'exemple 15 en solution dans 10 ml de méthanol sont traités par 0,8 g de soude et laissés sous agitation pendant 30 heures à température ambiante. Après le même traitement qu'à l'exemple 7, suivi d'une recristallisation dans l'éther diisopropylique, on isole 0, 51 g (86 %) du produit attendu fondant à 238-239°C; α D= -28,3° (c=1, DMF)

### Exemple 17:

Activité biologique du composé de l'exemple 4

| COMPOSE | Affinités pour les récepteurs Kd (nM) ^{(a)} | | | Activité antagoniste sur la différenciation des cellules F9 (IC₅₀, nM)^{(b)} |
|---|---|---|---|---|
| | RARα | RARβ | RARγ | |
| Exemple 4 | 380 | 19 | 17 | 40 |

| | | | | |
|---|---|---|---|---|
| (a) Les affinités pour les RARs sont déterminées dans les conditions décrites dans la publication : B. Martin et al.: Selective Synthetic Ligands for Human Nuclear Retinoic Acid Receptors. Skin Pharmacol. 1992; 5:57-65. | | | | |
| (b) L'activité antagoniste est déterminée en co-incubant un agoniste de référence (N-[4-Carboxybenzyl)-(5,6,7,8-tetrahydro-5,5,8,8-tetramethyl-2-naphtalenyl) amine (F9 AC₅₀ = 4nM) et différentes concentrations du rétinoïde à tester. Ces expériences sont effectuées dans les conditions de détermination de l'activité sur la différenciation de cellules F9 selon la publication ci-dessous : Darmon, M., M. Rocher, M.T. Cavey, B. Martin, T. Rabilloud, C. Delescluse and B. Shroot. Biological activity of retinoids correlates with affinity for nuclear receptors, but not for cytosolic binding protein. Skin Pharmacol. 1:161-175 (1988). | | | | |

### B. EXEMPLES DE FORMULATION

### 1) VOIE ORALE

### Exemple 18 :

On prépare la composition suivante sous la forme d'un comprimé de 0,8 g

| | |
|---|---|
| Composé de l'exemple 4 | 0,005 g |
| Amidon prégélatinisé | 0,265 g |
| Cellulose microcristalline | 0,300 g |
| Lactose | 0,200 g |
| Stéarate de magnésium | 0,030 g |

Pour le traitement de l'acné, on administre à un individu adulte 1 à 3 comprimés par jour pendant 3 à 6 mois selon la gravité du cas traité.

### Exemple 19 :

On prépare une suspension buvable, destinée à être conditionnée en ampoules de 5 ml

| | |
|---|---|
| Composé de l'exemple 4 | 0,050 g |
| Glycérine | 0,500 g |
| Sorbitol à 70 % | 0,500 g |
| Saccharinate de sodium | 0,010 g |
| Parahydroxybenzoate de méthyle Arôme q.s. | 0,040 g |
| Eau purifiée q.s.p | 5 ml |

Pour le traitement de l'acné, on administre à un individu adulte 1 ampoule par jour pendant 3 mois selon la gravité du cas traité.

### Exemple 20 :

On prépare la formulation suivante destinée à être conditionnée en gélules :

| | |
|---|---|
| Composé de l'exemple 4 | 0,025 g |
| Amidon de maïs | 0,060 g |
| Lactose q.s.p | 0,300 g |

Les gélules utilisées sont constituées de gélatine, d'oxyde de titane et d'un conservateur.

Dans le traitement du psoriasis, on administre à un individu adulte, 1 gélule par jour pendant 30 jours.

### 2) VOIE TOPIQUE

### Exemple 21 :

On prépare la crème Eau-dans l'Huile non ionique suivante :

| | |
|---|---|
| Composé de l'exemple 4 | 0,100 g |
| Mélange d'alcools de lanoline émulsifs, de cires et d'huiles raffinés, vendu par la Société BDF sous la dénomination "Eucérine anhydre" | 39,900 g |
| Parahydroxybenzoate de méthyle | 0,075 g |
| Parahydroxybenzoate de propyle | 0,075 g |
| Eau déminéralisée stérile q.s.p. | 100,000 g |

Cette crème est appliquée sur une peau psoriatique 1 à 2 fois par jour pendant 30 jours.

### Exemple 22 :

On prépare un gel en réalisant la formulation suivante :

| | |
|---|---|
| Composé de l'exemple 4 | 0,050 g |
| Erythromycine base | 4,000 g |
| Butylhydroxytoluène | 0,050 g |
| Hydroxypropylcellulose vendue par la société Hercules sous le nom de "KLUCEL HF" | 2,000 g |
| Ethanol (à 95°) q.s.p. | 100,000 g |

Ce gel est appliqué sur une peau atteinte de dermatose ou une peau acnéique 1 à 3 fois par jour pendant 6 à 12 semaines selon la gravité du cas traité.

### Exemple 23 :

On prépare une lotion antiséborrhéique en procédant au mélange des ingrédients suivants :

| | |
|---|---|
| Composé de l'exemple 4 | 0,030 g |
| Propylène glycol | 5,000 g |
| Butylhydroxytoluène | 0,100 g |
| Ethanol (à 95°) q.s.p | 100,000 g |

Cette lotion est appliquée deux fois par jour sur un cuir chevelu séborrhéique et on constate une amélioration significative dans un délai compris entre 2 et 6 semaines.

### Exemple 24 :

On prépare une composition cosmétique contre les effets néfastes du soleil en procédant au mélange des ingrédients suivants :

| | |
|---|---|
| Composé de l'exemple 5 | 1,000 g |
| Benzylidène camphre | 4,000 g |
| Triglycérides d'acides gras | 31,000 g |
| Monostéarate de glycérol | 6,000 g |
| Acide stéarique | 2,000 g |
| Alcool cétylique | 1,200 g |
| Lanoline | 4,000 g |
| Conservateurs | 0,300 g |
| Propylène glycol | 2,000 g |
| Triéthanolamine | 0,500 g |
| Parfum | 0,400 g |
| Eau déminéralisée q.s.p | 100,000 g |

Cette composition est appliquée quotidiennement, elle permet de lutter contre le vieillissement photoinduit.

### Exemple 25 :

On prépare la crème Huile dans l'Eau non ionique suivante :

| | |
|---|---|
| Composé de l'exemple 5 | 0,500 g |
| Vitamine D3 | 0,020 g |
| Alcool cétylique | 4,000 g |
| Monostéarate de glycérol | 2,500 g |
| Stéarate de PEG 50 | 2,500 g |
| Beurre de Karité | 9,200 g |
| Propylène glycol | 2,000 g |
| Parahydroxybenzoate de méthyle | 0,075 g |
| Parahydroxybenzoate de propyle | 0,075 g |
| Eau déminéralisée stérile q.s.p | 100,000 g |

Cette crème est appliquée sur une peau psoriatique 1 à 2 fois par jour pendant 30 Jours.

### Exemple 26 :

On prépare un gel topique en procédant au mélange des ingrédients suivants:

| | |
|---|---|
| Composé de l'exemple 13 | 0,050 g |
| Ethanol | 43,000 g |
| a-tocophérol | 0,050 g |
| Polymère carboxyvinylique vendu sous la dénomination "Carbopol 941" par la société "Goodrich | 0,500 g |
| Triéthanolamine en solution aqueuse à 20 % en poids | 3,800 g |
| Eau 9,300 g Propylène glycol qsp | 100,000 g |

Ce gel est appliqué dans le traitement de l'acné 1 à 3 fois par jour pendant 6 à 12 semaines selon la gravité du cas traité.

### Exemple 27 :

On prépare une lotion capillaire anti-chute et pour la repousse des cheveux en procédant au mélange des ingrédients suivants :

| | |
|---|---|
| Composé de l'exemple 13 | 0,05 g |
| Composé vendu sous la dénomination "Minoxidil" | 1,00 g |
| Propylène glycol | 20,00g |
| Ethanol | 34,92 g |
| Polyéthylèneglycol (masse moléculaire = 400) | 40,00 g |
| Butylhydroxyanisole | 0,01 g |
| Butylhydroxytoluène | 0,02 g |
| Eau qsp | 100,00 g |

On applique cette lotion 2 fois par jour pendant 3 mois sur un cuir chevelu ayant subi une chute de cheveu importante.

### Exemple 28 :

On prépare une crème anti-acnéique en procédant au mélange des ingrédients suivants :

| | |
|---|---|
| Composé de l'exemple 6 | 0,050 g |
| Acide rétinoïque | 0,010 g |
| Mélange de stéarates de glycérol et de polyéthylène glycol (75 moles) vendu sous le nom de "Gelot 64" par la société "GATTEFOSSE" | 15,000 g |
| Huile de noyau polyoxyéthylénée à 6 moles d'oxyde ... d'éthylène vendue sous le nom de "Labrafil M2130 CS" par la société "GATTEFOSSE" | 8,000 g |
| Perhydrosqualène | 10,000 g |
| Conservateurs | qs |
| Polyéthylèneglycol (masse moléculaire = 400) | 8,000 g |
| Sel disodique de l'acide éthyléne-diamine tétracétique | 0,050 g |
| Eau purifiée qsp | 100,00g |

Cette crème est appliquée sur une peau atteinte de dermatose ou une peau acnéique 1 à 3 fois par jour pendant 6 à 12 semaines.

### Exemple 29 :

On prépare une crème huile dans l'eau en réalisant la formulation suivante :

| | |
|---|---|
| Composé de l'exemple 7 | 0,020 g |
| 17-valérate de bétamethasone | 0,050 g |
| S-carboxyméthyl cystéine | 3, 000 g |
| Stéarate de polyoxyéthylène (40 moles d'oxyde d'éthylène) vendu sous le nom de "Myrj 52" par la société "ATLAS | 4,000 g |
| Monolaurate de sorbitan, polyoxyéthylène à 20 moles d'oxyde d'éthylène vendu sous le nom de "Tween 20" par la société "ATLAS" | 1,800 g |
| Mélange de mono et distéarate de glycérol vendu sous la dénomination de "Géléol" par la société "GATTEFOSSE" | 4,200 g |
| Propylène glycol | 10,000 g |
| Butylhydroxyanisole | 0,010 g |
| Butylhydroxytoluène | 0,020 g |
| Alcool cétostéarylique | 6,200 g |
| Conservateurs | q.s. |
| Perhydrosqualène | 18,000 g |
| Mélange de triglycérides caprylique-caprique vendu sous la dénomination de "Miglyol 812" par la société "DYNAMIT NOBEL" | 4,000 g |
| Triéthanolamine (99 % en poids) | 2,500 g |
| Eau q.s.p | 100,00g |

Cette crème est appliquée 2 fois par jour sur une peau atteinte de dermatose pendant 30 jours.

### Exemple 30 :

On prépare la crème de type huile dans l'eau suivante :

| | |
|---|---|
| Acide lactique | 5,000 g |
| Composé de l'exemple 12 | 0,020 g |
| Stéarate de polyoxyéthylène (40 moles d'oxyde d'éthylène) vendu sous le nom de "Myrj 52" par la société "ATLAS" | 4,000 g |
| Monolaurate de sorbitan, polyoxyéthylène à 20 moles d'oxyde d'éthylène vendu sous le nom de Tween 20" par la société "ATLAS" | 1,800 g |
| Mélange de mono et distéarate de glycérol vendu sous la dénomination de "Geleol" par la société "GATTEFOSSE" | 4,200 g |
| Propylène glycol | 10,000 g |
| Butylhydroxyanisole | 0,010 g |
| Butylhydroxytoluène | 0,020 g |
| Alcool cétostéarylique | 6,200 g |
| Conservateurs | q.s. |
| Perhydrosqualène | 18,000g |
| Mélange de triglycérides caprylique-caprique vendu sous la dénomination de "Miglyol 812" par la société "DYNAMIT NOBEL" | 4,000 g |
| Eau q.s.p | 100,00g |

Cette crème est appliquée 1 fois par jour, elle aide à lutter contre le vieillissement qu'il soit photoinduit ou chronologique.

## Revendications

1. Composés, caractérisés par le fait qu'ils présentent la formule (I) suivante : 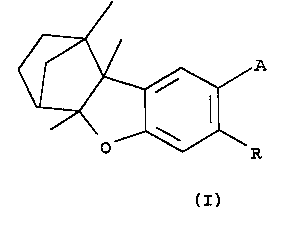 dans laquelle :
* R représente un atome d'hydrogène, un atome d'halogène, un radical alkyle ayant de 1 à 6 atomes de carbone, un radical acyle ayant de 1 à 10 atomes de carbone, un radical OR', R' ayant la signification donnée ci-après,
* A représente un radical choisi parmi les radicaux de formules (IIa-IIc) suivantes : 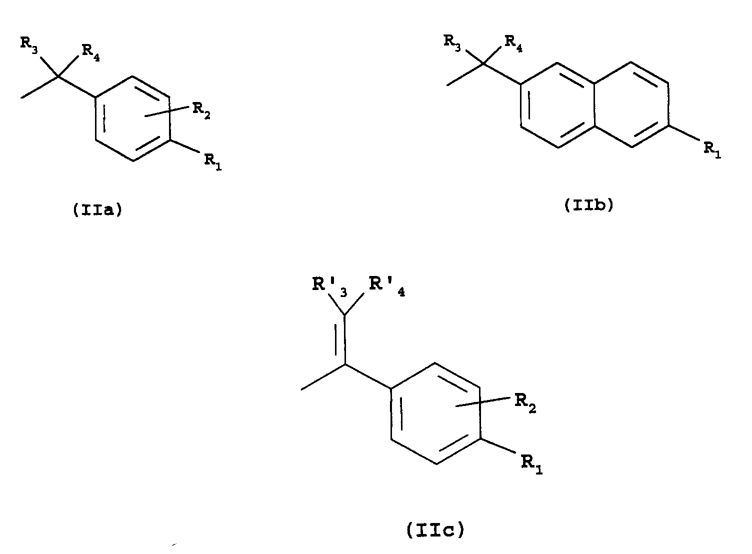 formules dans lesquelles :
- R₁ représente :
(i) un atome d'hydrogène,
(ii) le radical -CH₃,
(iii) le radical -CH₂-O-R_{5,}
(iv) un radical -OR₅
(v) un radical 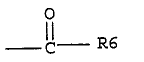
(vi) un radical -S(O)ₜ R₆
t, R₅ et R₆ ayant les significations données ci-après,
- R₂ représente un atome d'hydrogène ou le radical -OR₅
R₅ ayant la signification donnée ci-après,
- soit R₃ et R₄ indépendamment représentent un atome d'hydrogène, un radical alkyle inférieur ou le radical -(X)ₙ - (CH₂)ₘ - R₇,
X et R₇ ayant les significations données ci-après,
soit R₃ et R₄, pris ensemble, forment un groupe oxo (=O), thioxo (=S), oxime ou un dérivant de l'oxime (R₆-O-N=), epoxy, cycloalkyle éventuellement substitué par un atome d'halogène ou un radical alkyle inférieur, ou encore un groupe dioxolanne [-O-CH₂)_{q}-O-] avec q égal à 2 ou 3, R₆ ayant la signification donnée ci-après,
- R₃' et R₄' représentent indépendamment un atome d'hydrogène, un radical alkyle inférieur, un radical acyle inférieur,
étant entendu que dans tout ce qui précède :
. t est égal à 0, 1 ou 2,
. R₅ représentant un atome d'hydrogène, un radical alkyle inférieur, ou un radical acyle inférieur
. R₆ représente :
. un atome d'hydrogène,
. un radical -N (R'',R'''), R'' et R''' ayant les significations données ci-après,
. un radical -OR₈, R₈ ayant la signification donnée ci-après,
. R₇ représente un atome d'hydrogène, un radical -(C0)ₚ-R₉ dans lequel p peut avoir la valeur 0 ou 1, R₉ ayant la signification donnée ci-après,
. R₈ et R' représentent indépendamment un atome d'hydrogène, un radical alkyle ayant de 1 à 20 atomes de carbone, un radical mono ou polyhydroxyalkyle, un radical aryle éventuellement substitué, aralkyle éventuellement substitué, un reste de sucre ou un reste d'amino-acide,
. R₉ représente :
. un atome d'hydrogène,
. un radical alkyle,
. un radical alcenyl,
. un radical alcynyle,
. un radical aryle,
. un radical -OR₇ - dans ce cas, m ne peut prendre la valeur O, R₇ ayant la signifcation indiquée ci-dessus.
. un radical -N(R'', R'''), R'' et R''' ayant les significations données ci-après,
. R'' et R''', identiques ou différents, représentent un atome d'hydrogène, un radical alkyle inférieur, un radical mono ou polyhydroxyalkyle, un radical aryle éventuellement substitué, un radical aralkyle éventuellement substitué, un reste d'amino-acide ou de peptide ou de sucre ou encore R'' et R''' pris ensemble forment un hétérocycle,
. X est un atome d'oxygène ou de soufre,
. n varie de 0 à 1 et m de 0 à 10,
et les isomères optiques et géométriques des dits composés de formule (I), ainsi que leurs sels dans le cas où R₁ ou R₉ représente une fonction acide, ou lorsque R₉ représente une fonction amine.

2. Composés selon la revendication 1, caractérisés par le fait qu'ils se présentent sous forme de sels par addition d'une base ou d'un acide.

3. Composés selon l'une des revendications précédentes, caractérisés par le fait que le radical alkyle ayant de 1 à 6 atomes de carbone est choisi dans le groupe constitué par les radicaux méthyle, éthyle, isopropyle, butyle, tertiobutyle et hexyle.

4. Composés selon l'une des revendications précédentes, caractérisés par le fait que le radical acyle ayant de 1 à 10 atomes de carbone est choisi dans le groupe constitué par les radicaux acétyle, propionyle, pivaloyle.

5. Composés selon l'une des revendications précédentes, caractérisés par le fait que le radical monohydroxyalkyle est choisi dans le groupe constitué par les radicaux 2-hydroxyéthyle, 2-hydroxypropyle ou 3-hydroxypropyle.

6. Composés selon l'une des revendications précédentes, caractérisés par le fait que le radical polyhydroxyalkyle est choisi dans le groupe constitué par les radicaux 2,3-dihydroxypropyle, 2,3,4-trihydroxybutyle, 2,3,4,5-tétrahydroxypentyle ou le reste du pentaérythritol.

7. Composés selon l'une des revendications précédentes, caractérisés par le fait que le radical aryle est un radical phényle éventuellement substitué par un ou plusieurs atomes d'halogène, une fonction hydroxyle, nitro ou un groupe méthoxy.

8. Composés selon l'une des revendications précédentes, caractérisés par le fait que par radical aralkyle est le radical benzyle ou phénéthyle, éventuellement substitué par un ou plusieurs atomes d'halogène, une fonction hydroxyle ou nitro, ou un groupe méthoxy.

9. Composés selon l'une des revendications précédentes, caractérisés par le fait que le reste de sucre est un reste dérivant du glucose, du galactose, du mannose ou de l'acide glucuronique.

10. Composés selon l'une des revendications précédentes, caractérisés par le fait que l'hétérocycle est un radical pipéridino, morpholino, pyrrolidino ou pipérazino, éventuellement substitué en position 4 par un radical alkyle en C₁-C₆ ou mono ou polyhydroxyalkyle tels que définis ci-dessus.

11. Composés selon l'une quelconque des revendications précédentes, caractérisés par le fait qu'ils sont choisis dans le groupe constitué par :
[(1,2,3,4-Tétrahydro-1,4a,9b-triméthyl-1,4-méthanodibenzofuranne-8-yl) carbonyl]-6-naphtalène-2-carboxylate de méthyle
Acide [(1,2,3,4-Tétrahydro-1,4a,9b-triméthyl-1,4-méthanodibenzofuranne-8-yl) carbonyl]-6-naphtalène-2-carboxylique
Acide [(1,2,3,4-Tétrahydro-1,4a,9b-triméthyl-1,4-méthanodibenzofuranne-8-yl) méthyl]-6-naphtalène-2-carboxylique
[(1,2,3,4-Tétrahydro-1,4a,9b-triméthyl-1,4-méthanodibenzofuranne-8-yl) hydroxyméthyl]6-naphtalène-2-carboxylate de méthyle
Acide [(1,2,3,4-Tétrahydro-1,4a,9b-triméthyl-1,4-méthanodibenzofuranne-8-yl) hydroxyméthyl]-6-naphtalène-2-carboxylique
4-[(1,2,3,4-Tétrahydro-1,4a,9b-triméthyl-1,4-méthanodibenzofuranne-8-yl)-carbonyl] benzoate de méthyle
Acide 4-[(1,2,3,4-Tétrahydro-1,4a,9b-triméthyl-1,4-méthanodibenzofuranne-8-yl)-carbonyl] benzoïque
4-[(1,2,3,4-Tétrahydro-1,4a,9b-triméthyl-1,4-méthanodibenzofuranne-8-yl) hydroxyméthyl] phénylcarbinol
4-[(1,2,3,4-Tétrahydro-1,4a,9b-triméthyl-1,4-méthanodibenzofuranne-8-yl)-carbonyl] phénylcarboxaldéhyde
2-(1,2,3,4-Tétrahydro-1,4a,9b-triméthyl-1,4-méthanodibenzofuranne-8-yl)-2-(4-méthoxycarbonylphényl)-1,3-dioxolanne
2-(1,2,3,4-Tétrahydro-1,4a,9b-triméthyl-1,4-méthanodibenzofuranne-8-yl)-2-(4-carboxyphényl)-1,3-dioxolanne
4-[(1,2,3,4-tetrahydro-1,4a,7,9b-tetraméthyl-1,4-methanodibenzofuranne-8-yl) carbonyl] benzoate de méthyle
Acide 4-[(1,2,3,4-tetrahydro-1,4a,7,9b-tetraméthyl-1,4-methanodibenzofuranne-8-yl) carbonyl] benzoïque.

12. Composés selon la revendication 1, caractérisés par le fait qu'ils répondent à la formule générale (I), dans laquelle A représente le radical de formule (IIb).

13. Composés selon la revendication précédente, caractérisés en ce que R₁ représente le radical

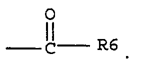

14. Composés intermédiaires, caractérisés en ce qu'ils répondent à la formule générale (X) suivante : 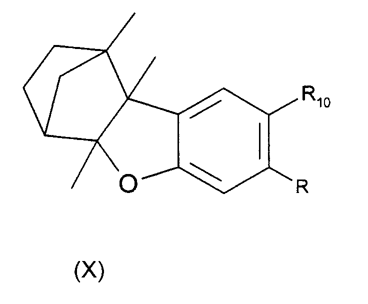 formule (X) dans laquelle R a les significations de R décrit dans la revendication 1, R₁₀ peut avoir les significations de R₁ décrit dans la revendication 1, à l'exception de l'atome d'hydrogène, ou peut également représenter un atome d'halogène, le radical -C(O)-R₈, ou le radical -C(O)-CH₂-halogène ou (CH₂)ₙ N (R''R'''), R₈ , R'' et R''' étant tels que définis dans la formule générale (I) selon la revendication 1.

15. Composés selon la revendication 14, caractérisés par la fait que'ils sont choisis dans le groupe constitué par :
1,2,3,4-Tétrahydro-1,4a,9b-triméthyl-1,4-méthanodibenzofuranne-8-yl méthylcétone;
8-(1,2,3,4-Tétrahydro-1,4a,9b-triméthyl-1,4-méthanodibenzofuranne)carboxaldéhyde;
Acide 8-(1,2,3,4-Tétrahydro-1,4a,9b-triméthyl-1,4-méthanodibenzofuranne) carboxylique;
1,2,3,4-Tétrahydro-1,4a,9b-triméthyl-1,4-méthanodibenzofuranne-8-yl, bromométhylcétone;
8-Ethynyl-1,2,3,4-tétrahydro-1,4a,9b-triméthyl-1,4-méthanodibenzofuranne;
1,2,3,4-Tetrahydro-1,4a,7,9b-tetraméthyl-1,4-methanodibenzofuranne

16. Médicament, caractérisé en ce qu'il consiste en l'un des composés définis selon l'une quelconque des revendications 1 à 13.

17. Composition pharmaceutique, caractérisée par le fait qu'elle contient dans un véhicule approprié, pour une administration par voie entérale, parentérale, topique ou oculaire, au moins un composé de formule (I) selon l'une quelconque des revendications 1 à 13.

18. Composition selon la revendication 17, caractérisée par le fait qu'elle contient de 0,001 à environ 5 % en poids d'un composé de formule (I).

19. Utilisation d'un composé selon l'une quelconque des revendications 1 à 13 pour la préparation d'une composition pharmaceutique destinée au traitement des affections dermatologiques, rhumatismales, respiratoires ainsi qu'ophtalmologiques.

20. Composition cosmétique pour l'hygiène corporelle et capillaire, caractérisée par le fait qu'elle contient, dans un véhicule cosmétique approprié, au moins un composé de formule (I) selon l'une quelconque des revendications 1 à 13.

21. Composition cosmétique selon la revendication 20, caractérisée par le fait qu'elle contient le composé de formule (I) à une concentration comprise entre 0,001 et 3 % en poids.

22. Utilisation des composés tels que définis dans l'une des revendications 14 et 15 pour la préparation des composés de formule (I) définis selon l'une des revendications 1 à 13.

## Claims

1. Compounds, characterized in that they have the following formula (I) : 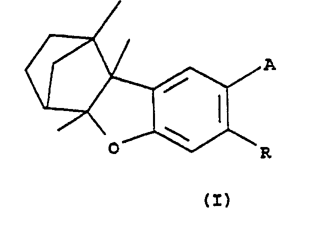 in which:
* R represents a hydrogen atom, a halogen atom, an alkyl radical having from 1 to 6 carbon atoms, an acyl radical having from 1 to 10 carbon atoms or an OR' radical, R' having the meaning given hereinbelow,
* A represents a radical chosen from the radicals of following formulae (IIa-IIc): 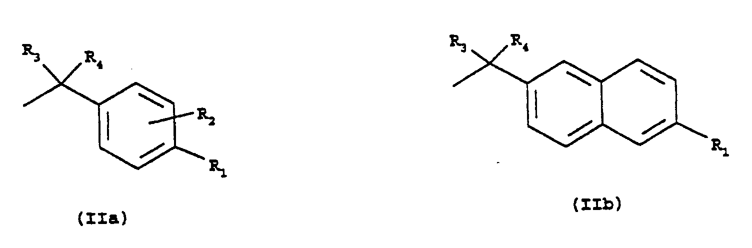 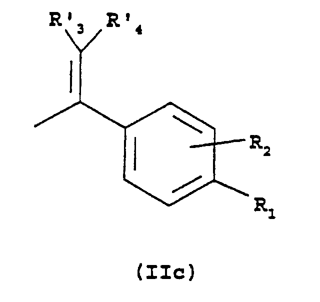 in which formulae:
- R₁ represents:
(i) a hydrogen atom,
(ii) the -CH₃ radical,
(iii) the -CH₂-O-R₅ radical,
(iv) an -OR₅ radical
(v) a 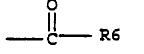 radical
(vi) an -S(O)ₜR₆ radical,
t, R₅ and R₆ having the meanings given hereinbelow,
- R₂ represents a hydrogen atom or the -OR₅ radical,
R₅ having the meaning given hereinbelow,
- either R₃ and R₄ independently represent a hydrogen atom, an alkyl radical having from 1 to 6 carbon atoms or the -(X)ₙ-(CH₂)ₘ-R₇ radical,
X and R₇ having the meanings given hereinbelow,
or R₃ and R₄, taken together, form an oxo (=O) group, a thioxo (=S) group, an oxime group or a group derived from the oxime (R₆-O-N=), an epoxy group, a cycloalkyl group optionally substituted by a halogen atom or an alkyl radical having from 1 to 6 carbon atoms, or alternatively a dioxolane group [-O-(CH₂)_{q}-O-] with q equal to 2 or 3, R₆ having the meaning given hereinbelow,
- R₃' and R₄' independently represent a hydrogen atom, an alkyl radical having from 1 to 6 carbon atoms or an acyl radical having from 1 to 10 carbon atoms,
it being understood that in all the above:
• t is equal to 0, 1 or 2,
• R₅ represents a hydrogen atom, an alkyl radical having from 1 to 6 carbon atoms or an acyl radical having from 1 to 10 carbon atoms,
• R₆ represents:
• a hydrogen atom,
• an -N(R'',R''') radical, R'' and R''' having the meanings given hereinbelow,
• an -OR₈ radical, R₈ having the meaning given hereinbelow,
• R₇ represents a hydrogen atom or a -(CO)ₚ-R₉ radical in which p can have the value 0 or 1, R₉ having the meaning given hereinbelow,
• R₈ represents a hydrogen atom, an alkyl radical having from 1 to 20 carbon atoms, a mono- or polyhydroxyalkyl radical, an aryl radical optionally substituted by one or more halogen atoms, a hydroxyl or nitro functional group, or a methoxy group; an aralkyl radical optionally substituted by one or more halogen atoms, a hydroxyl or nitro functional group, or a methoxy group; or a sugar residue,
• R' represents a hydrogen atom, an alkyl radical having from 1 to 20 carbon atoms, a mono- or polyhydroxyalkyl radical, an aryl radical optionally substituted by one or more halogen atoms, a hydroxyl or nitro functional group, or a methoxy group; or an aralkyl radical optionally substituted by one or more halogen atoms, a hydroxyl or nitro functional group, or a methoxy group,
• R₉ represents:
• a hydrogen atom,
• an alkyl radical,
• an alkenyl radical,
• an alkynyl radical,
• an aryl radical,
• an -OR₇ radical - in this case, m cannot take the value 0, R₇ having the meaning indicated above,
• an -N(R'',R''') radical, R'' and R''' having the meanings given hereinbelow,
• R'' and R''', which are identical or different, represent a hydrogen atom, an alkyl radical having from 1 to 6 carbon atoms, a mono- or polyhydroxyalkyl radical, an aryl radical optionally substituted by one or more halogen atoms, a hydroxyl or nitro functional group, or a methoxy group; an aralkyl radical optionally substituted by one or more halogen atoms, a hydroxyl or nitro functional group, or a methoxy group; or an amino acid or peptide or sugar residue or alternatively R'' and R''', taken together, form a heterocycle,
• X is an oxygen or sulphur atom,
• n varies from 0 to 1 and m from 0 to 10,
and the optical and geometrical isomers of the said compounds of formula (I), as well as their salts in the case where R₁ or R₉ represents an acid functional group or when R₉ represents an amine functional group.

2. Compounds according to Claim 1, characterized in that they are provided in the form of salts by addition of a base or of an acid.

3. Compounds according to either of the preceding claims, characterized in that the alkyl radical having from 1 to 6 carbon atoms is chosen from the group consisting of the methyl, ethyl, isopropyl, butyl, tert-butyl and hexyl radicals.

4. Compounds according to one of the preceding claims, characterized in that the acyl radical having from 1 to 10 carbon atoms is chosen from the group consisting of the acetyl, propionyl and pivaloyl radicals.

5. Compounds according to one of the preceding claims, characterized in that the monohydroxyalkyl radical is chosen from the group consisting of the 2-hydroxyethyl, 2-hydroxypropyl and 3-hydroxypropyl radicals.

6. Compounds according to one of the preceding claims, characterized in that the polyhydroxyalkyl radical is chosen from the group consisting of the 2,3-dihyroxypropyl, 2,3,4-trihydroxybutyl or 2,3,4,5-tetrahydroxypentyl radicals or the pentaerythritol residue.

7. Compounds according to one of the preceding claims, characterized in that the aryl radical is a phenyl radical optionally substituted by one or a number of halogen atoms, a hydroxyl or nitro functional group or a methoxy group.

8. Compounds according to one of the preceding claims, characterized in that the aralkyl radical is the benzyl or phenethyl radical, optionally substituted by one or a number of halogen atoms, a hydroxyl or nitro functional group or a methoxy group.

9. Compounds according to one of the preceding claims, characterized in that the sugar residue is a residue derived from glucose, galactose, mannose or glucuronic acid.

10. Compounds according to one of the preceding claims, characterized in that the heterocycle is a piperidino, morpholino, pyrrolidino or piperazino radical, optionally substituted in the 4-position by a C₁-C₆ alkyl radical or a mono- or polyhydroxyalkyl radical, such as defined above.

11. Compounds according to any one of the preceding claims, characterized in that they are chosen from the group consisting of:
Methyl 6-[(1,2,3,4-tetrahydro-1,4a,9b-trimethyl-1,4-methanodibenzofuran-8-yl)carbonyl]-2-naphthoate
6-[(1,2,3,4-Tetrahydro-1,4a,9b-trimethyl-1,4-methanodibenzofuran-8-yl)carbonyl]-2-naphthoic acid
6-[(1,2,3,4-Tetrahydro-1,4a,9b-trimethyl-1,4-methanodibenzofuran-8-yl)methyl]-2-naphthoic acid
Methyl 6-[(1,2,3,4-tetrahydro-1,4a,9b-trimethyl-1,4-methanodibenzofuran-8-yl)hydroxymethyl]-2-naphthoate
6-[(1,2,3,4-Tetrahydro-1,4a,9b-trimethyl-1,4-methanodibenzofuran-8-yl)hydroxymethyl]-2-naphthoic acid
Methyl 4-[(1,2,3,4-tetrahydro-1,4a,9b-trimethyl-1,4-methanodibenzofuran-8-yl)carbonyl]benzoate
4-[(1,2,3,4-Tetrahydro-1,4a,9b-trimethyl-1,4-methanodibenzofuran-8-yl)carbonyl]benzoic acid
4-[(1,2,3,4-Tetrahydro-1,4a,9b-trimethyl-1,4-methanodibenzofuran-8-yl)hydroxymethyl]phenylcarbinol
4-[(1,2,3,4-Tetrahydro-1,4a,9b-trimethyl-1,4-methanodibenzofuran-8-yl)carbonyl]benzaldehyde
2-(1,2,3,4-Tetrahydro-1,4a,9b-trimethyl-1,4-methanodibenzofuran-8-yl)-2-(4-methoxycarbonylphenyl)-1,3-dioxolane
2-(1,2,3,4-Tetrahydro-1,4a,9b-trimethyl-1,4-methanodibenzofuran-8-yl)-2-(4-carboxyphenyl)-1,3-dioxolane
Methyl 4-[(1,2,3,4-tetrahydro-1,4a,7,9b-tetramethyl-1,4-methanodibenzofuran-8-yl)carbonyl]-benzoate
4-[1,2,3,4-Tetrahydro-1,4a,7,9b-tetramethyl-1,4-methanodibenzofuran-8-yl)carbonyl]benzoic acid.

12. Compounds according to Claim 1, characterized in that they correspond to the general formula (I) in which A represents the radical of formula (IIb).

13. Compounds according to the preceding claim, characterized in that R₁ represents the 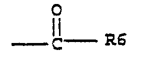 radical.

14. Intermediate compounds, characterized in that they correspond to the following general formula (X): 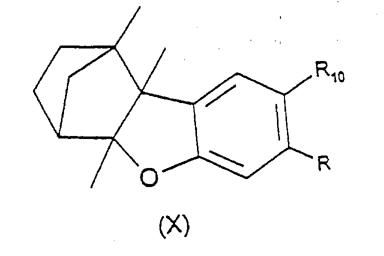 in which formula (X) R has the meanings of R described in Claim 1, R₁₀ can have the meanings of R₁ described in Claim 1, with the exception of the hydrogen atom, or can also represent a halogen atom, the -C(O)-R₈ radical or the -C(O)-CH₂-halogen or (CH₂)ₙN(R'',R''') radical, R₈, R'' and R''' being as defined in the general formula (I) according to Claim 1.

15. Compounds according to Claim 14, characterized in that they are chosen from the group consisting of:
1,2,3,4-Tetrahydro-1,4a,9b-trimethyl-1,4-methanodibenzofuran-8-yl methyl ketone;
1,2,3,4-Tetrahydro-1,4a,9b-trimethyl-1,4-methanodibenzofuran-8-carboxaldehyde;
1,2,3,4-Tetrahydro-1,4a,9b-trimethyl-1,4-methanodibenzofuran-8-carboxylic acid;
1,2,3,4-Tetrahydro-1,4a,9b-trimethyl-1,4-methanodibenzofuran-8-yl bromomethyl ketone;
8-Ethynyl-1,2,3,4-tetrahydro-1,4a,9b-trimethyl-1,4-methanodibenzofuran;
1,2,3,4-Tetrahydro-1,4a,7,9b-tetramethyl-1,4-methanodibenzofuran.

16. Medicament, characterized in that it is composed of one of the compounds defined according to any one of Claims 1 to 13.

17. Pharmaceutical composition, characterized in that it contains, in a vehicle appropriate for enteral, parenteral, topical or ocular administration, at least one compound of formula (I) according to any one of Claims 1 to 13.

18. Composition according to Claim 17, characterized in that it contains from 0.001 to approximately 5% by weight of a compound of formula (I).

19. Use of a compound according to any one of Claims 1 to 13 for the preparation of a pharmaceutical composition intended for the treatment of dermatological, rheumatic, respiratory and ophthalmological conditions.

20. Cosmetic composition for body and hair hygiene, characterized in that it contains, in an appropriate cosmetic vehicle, at least one compound of formula (I) according to any one of Claims 1 to 13.

21. Cosmetic composition according to Claim 20, characterized in that it contains the compound of formula (I) at a concentration of between 0.001 and 3% by weight.

22. Use of the compounds as defined in either of Claims 14 and 15 for the preparation of the compounds of formula (I) defined according to one of Claims 1 to 13.

## Patentansprüche

1. Verbindungen, dadurch gekennzeichnet, daß sie der folgenden allgemeinen Formel (I) entsprechen: 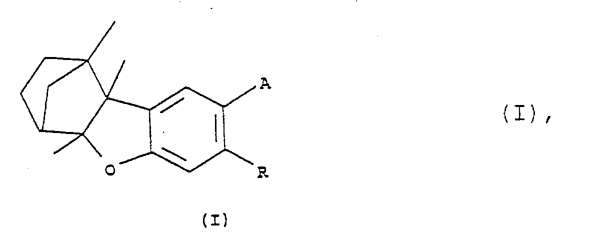 worin bedeuten:
- R ein Wasserstoffatom, ein Halogenatom, eine Alkylgruppe mit 1 bis 6 Kohlenstoffatomen, eine Acylgruppe mit 1 bis 10 Kohlenstoffatomen, eine Gruppe OR', wobei R' die nachstehend angegebenen Bedeutungen aufweist;
- A eine Gruppe, die unter den Gruppen der folgenden Formeln (IIa) bis (IIc) ausgewählt ist: 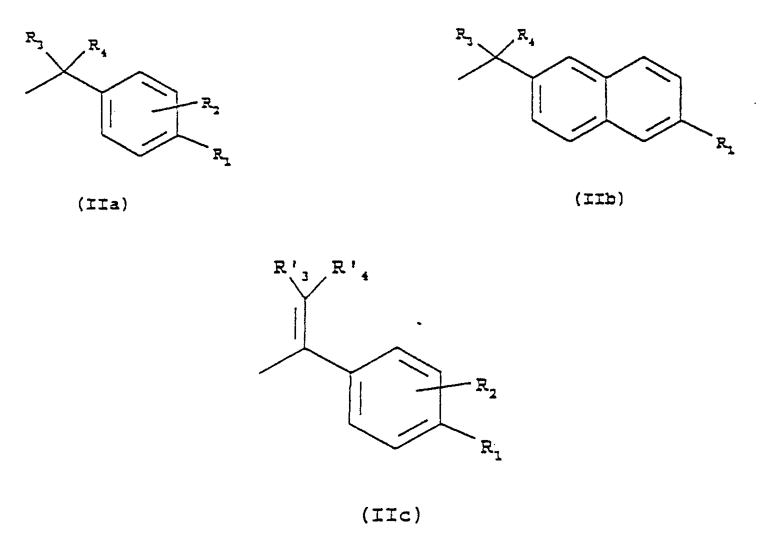 wobei in den Formeln:
- R₁ bedeutet:
(i) Wasserstoff,
(ii) die Gruppe -CH₃,
(iii) eine Gruppe -CH₂-O-R₅,
(iv) eine Gruppe -O-R₅,
(v) eine Gruppe 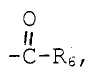
(vi) eine Gruppe -S(O)ₜ-R₆,
wobei t, R₅ und R₆ die nachstehend angegebenen Bedeutungen aufweisen,
- R₂ ein Wasserstoffatom oder eine Gruppe -OR₅ bedeutet, wobei R₅ die nachstehend angegebenen Bedeutungen aufweist,
- R₃ und R₄ entweder unabhängig voneinander ein Wasserstoffatom, eine Alkylgruppe mit 1 bis 6 Kohlenstoffatomen oder eine Gruppe -(X)ₙ-(CH₂)ₘ-R₇ bedeuten, wobei X und R₇ die nachstehend angegebenen Bedeutungen aufweisen, oder
R₃ und R₄ gemeinsam eine Oxogruppe (=O), eine Thioketongruppe (=S), ein Oxim oder ein Oximderivat (R₆-O-N=), eine Epoxygruppe, eine Cycloalkylgruppe, die gegebenenfalls mit einem Halogenatom oder einer Alkylgruppe mit 1 bis 6 Kohlenstoffatomen substituiert ist, oder auch eine Dioxolangruppe [-O-CH₂)_{q}-O-] bilden, wobei q 2 oder 3 bedeutet und R₆ die nachstehend angegebenen Bedeutungen aufweist,
- R₃' und R₄' unabhängig voneinander ein Wasserstoffatom, eine Alkylgruppe mit 1 bis 6 Kohlenstoffatomen oder eine Acylgruppe mit 1 bis 10 Kohlenstoffatomen,
mit der Maßgabe, daß bedeuten:
• t 0, 1 oder 2,
• R₅ Wasserstoff, eine Alkylgruppe mit 1 bis 6 Kohlenstoffatomen oder eine Acylgruppe mit 1 bis 10 Kohlenstoffatomen,
• R₆:
• ein Wasserstoffatom,
• eine Gruppe -N (R'', R'''), worin R'' und R''' die nachstehend angegebenen Bedeutungen aufweisen,
• eine Gruppe -OR₈, wobei R₈ die nachstehend angegebenen Bedeutungen aufweist,
• R₇ ein Wasserstoffatom, eine Gruppe -(CO)ₚ-R₉, worin p 0 oder 1 bedeuten kann und R₉ die nachfolgend angegebenen Bedeutungen aufweist,
• R₈ ein Wasserstoffatom, eine Alkylgruppe mit 1 bis 20 Kohlenstoffatomen, eine Mono- oder Polyhydroxyalkylgruppe, eine Arylgruppe, die gegebenenfalls mit einem oder mehreren Halogenatomen, einer Hydroxygruppe, einer Nitrogruppe oder einer Methoxygruppe substituiert ist, eine Aralkylgruppe, die gegebenenfalls mit einem oder mehreren Halogenatomen, einer Hydroxygruppe, einer Nitrogruppe oder einer Methoxygruppe substituiert ist, oder einen Zuckerrest,
• R' ein Wasserstoffatom, eine Alkylgruppe mit 1 bis 20 Kohlenstoffatomen, eine Mono- oder Polyhydroxyalkylgruppe, eine Arylgruppe, die gegebenenfalls mit einem oder mehreren Halogenatomen, einer Hydroxygruppe, einer Nitrogruppe oder einer Methoxygruppe substituiert ist, eine Aralkylgruppe, die gegebenenfalls mit einem oder mehreren Halogenatomen, einer Hydroxygruppe, einer Nitrogruppe oder einer Methoxygruppe substituiert ist,
• R₉:
• ein Wasserstoffatom,
• eine Alkylgruppe,
• eine Alkenylgruppe,
• eine Alkinylgruppe,
• eine Arylgruppe,
• eine Gruppe -OR₇, wobei in diesem Fall m nicht den Wert Null annehmen kann und R₇ die oben angegebenen Bedeutungen aufweist,
• eine Gruppe -N (R'', R'''), worin R'' und R''' die nachfolgend angegebenen Bedeutungen aufweisen,
• R'' und R''', die identisch oder voneinander verschieden sind, Wasserstoff, eine Alkylgruppe mit 1 bis 6 Kohlenstoffatomen, eine Mono- oder Polyhydroxyalkylgruppe, eine Arylgruppe, die gegebenenfalls mit einem oder mehreren Halogenatomen, einer Hydroxygruppe, einer Nitrogruppe oder einer Methoxygruppe substituiert ist, eine Aralkylgruppe, die gegebenenfalls mit einem oder mehreren Halogenatomen, einer Hydroxygruppe, einer Nitrogruppe oder einer Methoxygruppe substituiert ist, oder einen Aminosäure-, Petid- oder Zuckerrest oder R'' und R''' bilden gemeinsam einen Heterocyclus,
• X ein Sauerstoff- oder Schwefelatom, und
• n liegt im Bereich von 0 bis 1 und m im Bereich von 0 bis 10;
und die optischen und geometrischen Isomere der Verbindungen der Formel (I) sowie die Salze dieser Verbindungen, wenn R₁ oder R₉ eine Säuregruppe bedeuten oder wenn R₉ eine Aminogruppe bedeutet.

2. Verbindungen nach Anspruch 1, dadurch gekennzeichnet, daß sie durch Zugabe einer Säure oder einer Base in Salzform vorliegen.

3. Verbindungen nach einem der vorhergehenden Ansprüche, dadurch gekennzeichnet, daß die Alkylgruppe mit 1 bis 6 Kohlenstoffatomen unter Methyl, Ethyl, Isopropyl, Butyl, *tert*.-Butyl und Hexyl ausgewählt ist.

4. Verbindungen nach einem der vorhergehenden Ansprüche, dadurch gekennzeichnet, daß die Acylgruppe mit 1 bis 10 Kohlenstoffatomen unter Acetyl, Propionyl und Pivaloyl ausgewählt ist.

5. Verbindungen nach einem der vorhergehenden Ansprüche, dadurch gekennzeichnet, daß die Monohydroxyalkylgruppe unter 2-Hydroxyethyl, 2-Hydroxypropyl oder 3-Hydroxypropyl ausgewählt ist.

6. Verbindungen nach einem der vorhergehenden Ansprüche, dadurch gekennzeichnet, daß die Polyhydroxyalkylgruppe unter 2,3-Dihydroxypropyl, 2,3,4-Trihydroxybutyl, 2,3,4,5-Tetrahydroxypentyl oder der Pentaerythritgruppe ausgewählt ist.

7. Verbindungen nach einem der vorhergehenden Ansprüche, dadurch gekennzeichnet, daß die Arylgruppe eine Phenylgruppe ist, die gegebenenfalls mit einem oder mehreren Halogenatomen, einer Hydroxygruppe, einer Nitrogruppe oder einer Methoxygruppe substituiert ist.

8. Verbindungen nach einem der vorhergehenden Ansprüche, dadurch gekennzeichnet, daß die Aralkylgruppe unter den Benzyl- oder Phenethylgruppen ausgewählt ist, die gegebenenfalls mit einem oder mehreren Halogenatomen, einer Hydroxygruppe, einer Nitrogruppe oder einer Methoxygruppe substituiert sind.

9. Verbindungen nach einem der vorhergehenden Ansprüche, dadurch gekennzeichnet, daß der Zuckerrest unter den Resten von Glucose, Galactose, Mannose oder Glucuronsäure ausgewählt ist.

10. Verbindungen nach einem der vorhergehenden Ansprüche, dadurch gekennzeichnet, daß die heterocyclischen Gruppen unter den Gruppen Piperidino, Morpholino, Pyrrolidino oder Piperazino ausgewählt sind, die gegebenenfalls in 4-Stellung mit einer C₁₋₆-Alkylgruppe oder Mono- oder Polyhydroxyalkylgruppe substituiert sind.

11. Verbindungen nach einem der vorhergehenden Ansprüche, dadurch gekennzeichnet, daß sie ausgewählt sind unter:
Methyl-[(1,2,3,4-Tetrahydro-1,4a,9b-trimethyl-1,4-methanodibenzofuran-8-yl)carbonyl)]-6-naphthalin-2-carboxylat,
[(1,2,3,4-Tetrahydro-1,4a,9b-trimethyl-1,4-methanodibenzofuran-8-yl)carbonyl)]-6-naphthalin-2-carbonsäure,
[(1,2,3,4-Tetrahydro-1,4a,9b-trimethyl-1,4-methanodibenzofuran-8-yl)methyl)]-6-naphthalin-2-carbonsäure,
Methyl-[(1,2,3,4-Tetrahydro-1,4a,9b-trimethyl-1,4-methanodibenzofuran-8-yl)hydroxymethyl)]-6-naphthalin-2-carboxylat,
[(1,2,3,4-Tetrahydro-1,4a,9b-trimethyl-1,4-methanodibenzofuran-8-yl)hydroxymethyl)-6-naphthalin-2-carbonsäure,
Methyl-4-[(1,2,3,4-Tetrahydro-1,4a,9b-trimethyl-1,4-methano-dibenzofuran-8-yl)carbonyl))-benzoat,
4-[(1,2,3,4-Tetrahydro-1,4a,9b-trimethyl-1,4-methanodibenzofuran-8-yl)carboyl)]-benzoesäure,
4-[(1,2,3,4-Tetrahydro-1,4a,9b-trimethyl-1,4-methanodibenzofuran-8-yl)hydroxymethyl)]-phenylcarbinol,
4-[(1,2,3,4-Tetrahydro-1,4a,9b-trimethyl-1,4-methanodibenzofuran-8-yl)carbonyl)]-phenylcarboxaldehyd,
2-(1,2,3,4-Tetrahydro-1,4a,9b-trimethyl-1,4-methanodibenzofuran-8-yl)-2-(4-methoxycarbonylphenyl)-1,3-dioxolan,
2-(1,2,3,4-Tetrahydro-1,4a,9b-trimethyl-1,4-methanodibenzofuran-8-yl)-2-(4-carboxyphenyl)-1,3-dioxolan,
Methyl-4-[(1,2,3,4-Tetrahydro-1,4a,7,9b-tetramethyl-1,4-methano-dibenzofuran-8-yl)carbonyl)]-benzoat, und
4-[(1,2,3,4-Tetrahydro-1,4a,7,9b-tetramechyl-1,4-methanodibenzofuran-8-yl)carbonyl)]-benzoesäure.

12. Verbindungen nach Anspruch 1, dadurch gekennzeichnet, daß sie der allgemeinen Formel (I) entsprechen, worin A die Gruppe der Formel (IIb) bedeutet.

13. Verbindungen nach dem vorhergehenden Anspruch, dadurch gekennzeichnet, daß R₁ die Gruppe 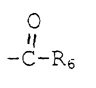 bedeutet.

14. Zwischenprodukte, dadurch gekennzeichnet, daß sie der folgenden allgemeinen Formel (X) entsprechen: 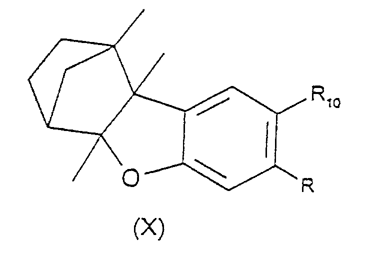 wobei in Formel (X) R die Bedeutungen von R gemäß Anspruch 1 aufweist und wobei R₁₀ die in Anspruch 1 angegebenen Bedeutungen von R₁ mit Ausnahme von Wasserstoff aufweisen kann und ferner auch ein Halogenatom, eine Gruppe -C(O)-R₈, eine Gruppe -C(O)-CH₂-Halogen oder eine Gruppe (CH₂)ₙN(R''R''') bedeuten kann, wobei R₈, R'' und R''' die Bedeutungen gemäß der in der allgemeinen Formel (I) in Anspruch 1 angegebenen Definition aufweisen.

15. Verbindungen nach Anspruch 14, dadurch gekennzeichnet, daß sie ausgewählt sind unter:
1,2,3,4-Teranydro-1,4a,9b-timethyl-1,4-methanodibenzofuran-8-yl-methylketon,
8-(1,2,3,4-Tetrahydro-1,4a,9b-trimethyl-1,4-methanodibenzofuran)carboxaldehyd,
1,2,3,4-tetrahydro-1,4a,7,9b-tétramethyl-1,4-methanodibenzofuran,
8-(1,2,3,4-Tetrahydro-1,4a,9b-trimethyl-1,4-methanodibenzofuran)carbonsäure,
1,2,3,4-Tetrahydro-1,4a,9b-trimethyl-1,4-methanodibenzofuran-8-yl-brommethylketon, und
8-Ethinyl-1,2,3,4-tetrahydro-1,4a,9b-trimethyl-1,4-methanodibenzofuran.

16. Arzneimittel, dadurch gekennzeichnet, daß es aus einer Verbindung nach einem der Ansprüche 1 bis 13 besteht.

17. Pharmazeutische Zusammensetzung, dadurch gekennzeichnet, daß sie in einem für eine enterale, parenterale, topische oder okulare Verabreichung geeigneten Träger mindestens eine Verbindung der Formel (I) nach einem der Ansprüche 1 bis 13 enthält.

18. Zusammensetzung nach Anspruch 17, dadurch gekennzeichnet, daß sie 0,001 bis etwa 5 Gew.-% einer Verbindung der Formel (I) enthält.

19. Verwendung einer Verbindung nach einem der Ansprüchen 1 bis 13 zur Herstellung einer pharmazeutischen Zusammensetzung, die zur Behandlung von dermatologischen Erkrankungen, rheumatischen Erkrankungen, Erkrankungen der Atemwege und ophthalmologischen Erkrankungen bestimmt ist.

20. Kosmetische Zusammensetzung zur Körper- und Haarhygiene, dadurch gekennzeichnet, daß sie in einem kosmetisch akzeptablen Träger mindestens eine Verbindung der Formel (I) nach einem der Ansprüche 1 bis 13 enthält.

21. Kosmetische Zusammensetzung nach Anspruch 20, dadurch gekennzeichnet, daß sie die Verbindung der Formel (I) in einer Konzentration im Bereich von 0,001 bis 3 Gew.-% enthält.

22. Verwendung der Verbindungen nach einem der Ansprüche 14 und 15 zur Herstellung der Verbindungen der Formel (I) nach einem der Ansprüche 1 bis 13.
